# EUROPEAN PATENT SPECIFICATION

(11) **EP 0 461 554 B1**
(45) Date of publication and mention of the grant of the patent: **05.01.1994**
(21) Application number: 91109350.8
(22) Date of filing: 07.06.1991
(51) Int. Cl.: C10L 1/18, C07C 69/017, C07C 43/20

(54) **Compositions of refinery hydrocarbons having improved fluidity at low temperatures**
Raffineriekohlenwasserstoffzusammensetzungen, eine verbesserte Tieftemperaturfliessfähigkeit besitzend
Compositions d'hydrocarbures de raffinerie ayant une fluidité améliorée à basses températures

(30) Priority: 08.06.1990 IT 2059690
(43) Date of publication of application: 18.12.1991
(73) Proprietor: SOCIETA' ITALIANA ADDITIVI PER CARBURANTI S.r.l. - S.I.A.C., 65100 Pescara (IT)
(72) Inventor: Santoro, Ettore, I-20037 Paderno Dugnano, Milan (IT); Canova, Luciano, Dr., I-28100 Novara (IT); Dalcanale, Enrico, Dr., I-43100 Parma (IT); Bonsignore, Stefanio, I-28100 Novara (IT); Falchi, Paolo, I-66100 Chieti (IT)
(74) Representative: Weinhold, Peter, Dr.

(56) References cited:
- EP-A- 0 300 800
- EP-A- 0 311 452
- EP-A- 0 339 826
- EP-A- 0 350 842
- EP-A- 0 400 773
- EP-A- 0 409 403
- DD-A- 264 445
- DD-A- 264 446
- GB-A- 1 167 427
- US-A- 2 034 024
- US-A- 2 801 926

## Description

The present invention relates to compositions based on liquid refinery hydrocarbons, such as gas oils and fuel oil and, more in general, products known as Middle Distillate which, upon lowering the temperature, undergo undesired physical changes which may be detected by measuring the following parameters: Cloud Point (C.P.), Pour Point (P.P.) and Cold Filter Plugging Point (C.F.P.P.) as defined, respectively, by ASTM-D 2500-81 and 97-85 and IP 309/83.

The term "Middle Distillate" denotes hydrocarbon fractions having a distillation range of from 150 to 450°C (ASTM-D 86-67).

It is well-known, for example, that the fluidity of gas oils used for motor vehicles, for naval and aircraft engines or for heating, decreases with decreasing temperature, thereby causing quite serious problems.

The above phenomenon is mainly due to the precipitation of n-paraffins contained in the gas oil.

It is also well-known that such problems can be overcome by the addition of suitable products, normally polymeric substances, to the above hydrocarbons.

The additives normally used for this purpose are ethylene/vinylacetate copolymers of a particular molecular weight and composition, or ethylene/propylene/unconjugated diene copolymers and terpolymers, prepared by using homogeneous catalysts (based on organometallic vanadium and aluminium compounds) as described in Italian patents No. 811,873 and 866,519.

US-A-3,374,073 and 3,756,954 propose as additives ethylene/propylene/conjugated or unconjugated diene terpolymers, prepared by homogeneous catalysis and subsequently degraded by thermal oxidation to certain molecular weights.

The most common additives for gas oil or Middle Distillate allow the use of these hydrocarbons also at low temperatures in that, by interfering with the crystallization kinetics, they determine the form and dimensions of the crystals and also limit the crystallization and the agglomeration of crystals, thus improving the liquid flow. Said additives, therefore, do not change the cloud point of the hydrocarbon compounds, i.e. the temperature of the first appearance of paraffin crystals in the liquid.

There has now been found a number of additives for the Middle Distillate which lower the cloud Point of said hydrocarbon fractions in that they inhibit the formation of crystalline nuclei of paraffins. Said nuclei, therefore, are formed at a lower temperature than in the case of paraffins without said additives.

Therefore, the present invention provides compositions of refinery hydrocarbons which comprise at least one additive which lowers the Cloud Point and has the general formula (I): 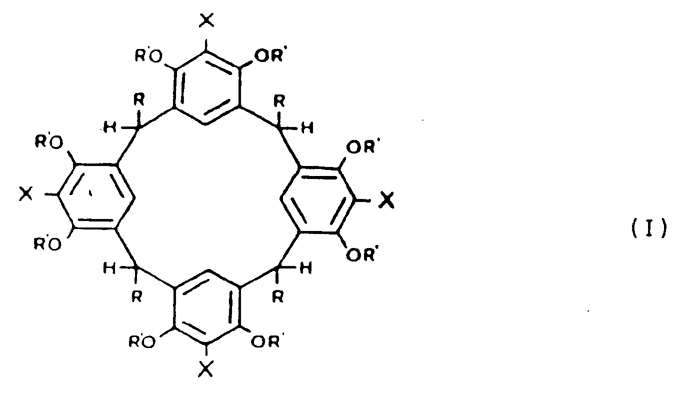
wherein R is hydrogen or a C₁-C₃₀ alkyl, iso-alkyl, cyclo-alkyl, aryl or alkyl-aryl group, the R' radicals, alike or different from each other, represent C₁-C₃₀ alkyl, iso-alkyl, cyclo-alkyl, aryl or alkyl-aryl groups or at least one of said radicals has one of the general formulae (II) to (IV):

-COR₁ (II)

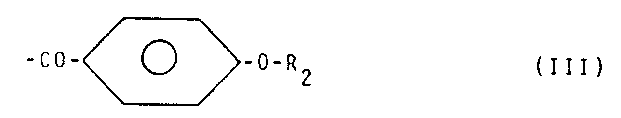

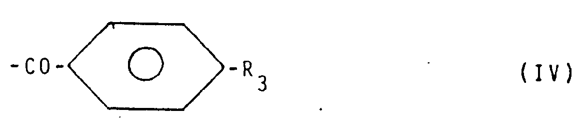

where R₁, R₂, R₃, alike or different from each other, represent C₁-C₃₀ alkyl, iso-alkyl, cyclo-alkyl, aryl or alkyl-aryl groups; and X is hydrogen, lower alkyl, preferably C₁-C₄ alkyl, or -OR', R' being defined as above.

Preferably the individual groups R, R' and X present in a certain compound all have the same meaning. For example, all groups X are identical, all groups R are the same, etc.

Some compounds covered by general formula I are already known from EP-A-350 842 and 300 800. However, said documents only disclose the potential use thereof in memory devices, opto-electronics and the like. EP-A-339 826 discloses some of the compounds of formula I as part of polymetallocene macrocycles which are reported to be potentially useful as electron transfer mediators, for chemical sensor design, as redox catalysts or as Second Harmonic Generator materials.

Preferred compounds of general formula I are those wherein X is selected from hydrogen, C₈-C₂₀ alkoxy and C₁-C₄ alkyl, particularly from hydrogen, methyl and C₁₀-C₁₈ alkoxy; and/or R is selected from hydrogen and C₁-C₂₀ alkyl, particularly from hydrogen, methyl and ethyl; and/or R' is selected from C₁-C₂₀ alkyl and groups of general formula (II) wherein R₁ is C₁-C₂₀ alkyl, particularly C₉-C₂₀ alkyl.

Examples of the groups mentioned in connection with general formula (I) are:
C₁-C₃₀ alkyl, preferably C₁-C₂₀ alkyl, such as methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl and nonadecyl;
C₃-C₃₀ isoalkyl, preferably C₃-C₂₀ isoalkyl, such as the isomeric forms of the above n-alkyl groups, e.g., isopropyl, isobutyl, sec-butyl, isooctyl etc.;
C₃-C₂₀, preferably C₃-C₁₀ cycloalkyl, such as cyclopropyl, cyclobutyl, cyclopentyl, cyclohexyl, cycloheptyl and cyclooctyl;
C₆-C₂₀, preferably C₆-C₁₄ aryl, such as phenyl, naphthyl, anthryl, biphenylyl etc.;
C₇-C₂₀, preferably C₇-C₁₅ alkyl-aryl, such as benzyl, phenethyl, naphthylmethyl, tolyl and xylyl.

Specific examples of compounds of general formula (I) which are particularly suitable as additives for lowering the Cloud Point of refinery hydrocarbons are:
3,5,10,12,17,19,24,26-octa-octadecanoyloxy-r-1,c-8,c-15,c-22-tetrahenaicosan-[l₄]metacyclophane;
3,4,5,10,11,12,17,18,19,24,25,26-dodeca-hexadecyloxy-r-1,c-8,c-15,c-22-tetramethyl-[l₄]metacyclophane;
3,4,5,10,11,12,17,18,19,24,25,26-dodeca-octadecanoyloxy-r-1,c-8,c-15,c-22-tetraundecyl-[l₄]metacyclophane;
4,11,18,25-tetramethyl-3,5,10,12,17,19,24,26-octadodecanoyloxy-[l₄]metacyclophane;
4,11,18,25-tetramethyl-3,5,10,12,17,19,24,26-octaoctadecanoyloxy-[l₄]metacyclophane;
3,5,10,12,17,19,24,26-octa-acetyloxy-r-1,c-8,c-15,c-22-tetraheptadecyl-[l₄]metacyclophane;
3,5,10,12,17,19,24,26-octa-octadecanoyloxy-r-1,c-8,c-15,c-22-tetramethyl-[l₄]metacyclophane;
3,5,10,12,17,19,24,26-octa-dodecanoyloxy-r-1,c-8,c-15,c-22-tetramethyl-[l₄]metacyclophane;
3,5,10,12,17,19,24,26-octa-tetradecanoyloxy-r-1,c-8,c-15,c-22-tetramethyl-[l₄]metacyclophane;
3,5,10,12,17,19,24,26-octa-p-dodecyloxybenzoyloxy-r-1,c-8,c-15,c-22-tetramethyl-[l₄]metacyclophane;
3,5,10,12,17,19,24,26-octa-hexanoyloxy-r-1,c-8,c-15,c-22-tetraethyl-[l₄]metacyclophane;
3,5,10,12,17,19,24,26-octa-decanoyloxy-r-1,c-8,c-15,c-22-tetraethyl-[l₄]metacyclophane;
3,4,5,10,11,12,17,18,19,24,25,26-dodeca-tridecanoyloxy-r-1,c-8,c-15,c-22-tetramethyl-[l₄]metacyclophane;
3,4,5,10,11,12,17,18,19,24,25,26-dodeca-tetradecanoyloxy-r-1,c-8,c-15,c-22-tetramethyl-[l₄]metacyclophane;
3,4,5,10,11,12,17,18,19,24,25,26-dodeca-hexadecanoyloxy-r-1,c-8,c-15,c-22-tetramethyl-[l₄]metacyclophane;
3,4,5,10,11,12,17,18,19,24,25,26-dodeca-octadecanoyloxy-r-1,c-8,c-15,c-22-tetramethyl-[l₄]metacyclophane;
3,4,5,10,11,12,17,18,19,24,25,26-dodeca-dodecanoyloxy-r-1,c-8,c-15,c-22-tetraethyl-[l₄]metacyclophane;
3,4,5,10,11,12,17,18,19,24,25,26-dodeca-decanoyloxy-r-1,c-8,c-15,c-22-tetraethyl-[l₄]metacyclophane, etc.

The tetramers having formula (I) can, e.g., be synthesized by reaction of resorcin, optionally substituted in position 2, with an aldehyde and subsequent esterification of the product obtained. The reaction between resorcin and aldehyde takes place according to the following reaction scheme: 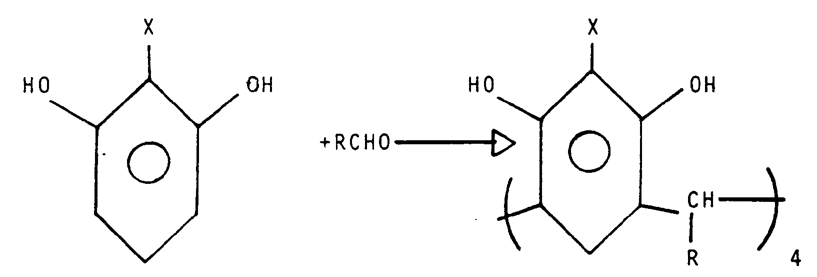
where X and R have the meanings given above. The esterification is preferably effected with organic acid halides or alkyl halides.

Examples of suitable resorcins are resorcinol, pyrogallol, 2,6-di-hydroxy-toluene, etc.

Preferred examples of aldehydes which can be used in the synthesis of the products of the present invention are formaldehyde, acetaldehyde, laurylaldehyde, enanthic aldehyde, benzaldehyde, 1-docosanal, stearic aldehyde, palmitic aldehyde, 1-decanal, etc.

The synthesis is generally carried out at room temperature, in a solvent, preferably methanol or ethanol, and in the presence of an acid catalyst.

Examples of suitable catalysts are hydrogen chloride, sulphuric acid, phosphoric acid, para-toluenesulfonic acid, etc., optionally diluted with water to a concentration of from 25 to 50% by weight.

These catalysts are generally used in amounts of from 1 to 20% by weight of the total weight of reagents.

The products thus obtained, which may be recovered from the solvent by means of well-known techniques, such as evaporation or precipitation by means of non-solvents, may then be esterified either with at least one halide having the formula R'-Y, where R' has the above-mentioned meaning and Y is halogen such as chlorine or bromine (particularly Cl), or with an acid anhydride (e.g. acetanhydride).

Examples of useful halides are acetyl chloride, lauroyl chloride, palmitoyl chloride, stearoyl chloride, myristoyl chloride, decanoyl chloride, tridecanoyl chloride, 1-bromohexadecane, 1-bromodecane, 1-bromododecane, etc.

In particular, the reaction with a compound of formula R'-Y, where R' is a C₁-C₃₀ alkyl, iso-alkyl, cycloalkyl, aryl or alkyl-aryl group, is preferably carried out in an organic solvent, such as dimethylformamide (DMF) or dimethylsulfoxide (DMSO) and in the presence of an organic base such as potassium carbonate.

Among the compounds of general formula (I), the substituted [l₄]-metacyclophanes of general formula (V): 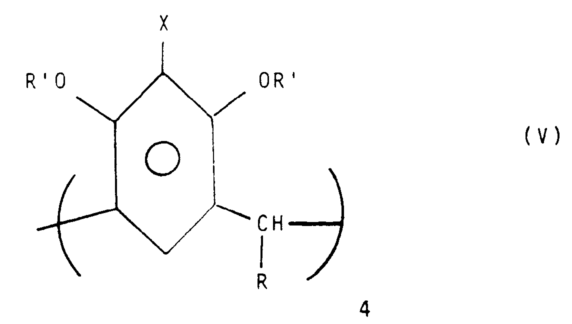
wherein X is
i) hydrogen and at the same time R is a linear C₄-C₃₀ alkyl or a C₃-C₃₀ iso-alkyl, cycloalkyl, aryl or alkyl-aryl group, and the radicals R', alike or different from each other, represent groups of general formulae (II) to (IV)

   -COR₁ (II)

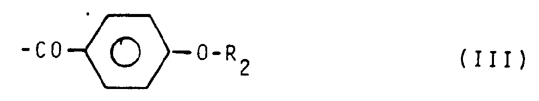

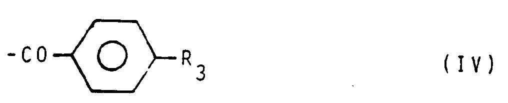

   where R₁, R₂ and R₃, alike or different from each other, are C₁-C₃₀ alkyl, iso-alkyl, cyclo-alkyl, aryl and alkyl-aryl groups;
ii) lower alkyl, such as C₁-C₄ alkyl, and at the same time R is a hydrogen or C₁-C₃₀ alkyl, iso-alkyl, cycloalkyl, aryl or alkyl-aryl and R' represents groups of general formulae (II), (III) and (IV), where R₁, R₂ and R₃, alike or different from each other, are C₁-C₃₀ alkyl, iso-alkyl, cyclo-alkyl, aryl or alkyl-aryl groups;
iii) a group OR' and at the same time R is C₃-C₃₀ alkyl, isoalkyl, cyclo-alkyl, aryl or alkyl-aryl and R' represents groups of general formulae (II), (III) and (IV) as defined hereinabove;
iv) a group OR'' and at the same time R is C₁-C₃₀ alkyl, isoalkyl, cyclo-alkyl, aryl or alkyl-aryl and R' and R'', the same, are C₁-C₃₀ alkyl, iso-alkyl, cyclo-alkyl, aryl or alkyl-aryl groups;

are new products. These products can also be obtained by using the methods described above.

The additives of general formula (I) can be added to the gas oils or to the hydrocarbon fractions known as Middle Distillate either as such or dissolved in a suitable solvent, preferably high-boiling aromatic hydrocarbons, such as the products commercially available under the trade-names SHELLSOL® AB, SHELLSOL ® A, E, R, SOLVESSO ® 100, 150, 200, HAN, etc. Other additives such as antioxidants, basic detergents, rust inhibitors, pour-point depressants, etc. may also be included in the compositions of the present invention.

The additives of general formula (I) are generally added to the hydrocarbon fractions in amounts of at least 0.001% by weight, preferably of from 0.001 to 1%, particularly of from 0.005 to 0.15% by weight.

The hydrocarbon fractions containing the compounds of general formula (I) generally show a decrease in the cloud point of at least 10°C.

It is to be noted that the above compounds inhibit the sedimentation of paraffins during storage as well as the corrosion of metal surfaces in contact with the hydrocarbon fractions.

The following examples are to illustrate the present invention without limiting it in any way.

To evaluate the anti-cloud properties of the present compounds, hydrocarbon fractions having the characteristics shown in Table 1 were examined.

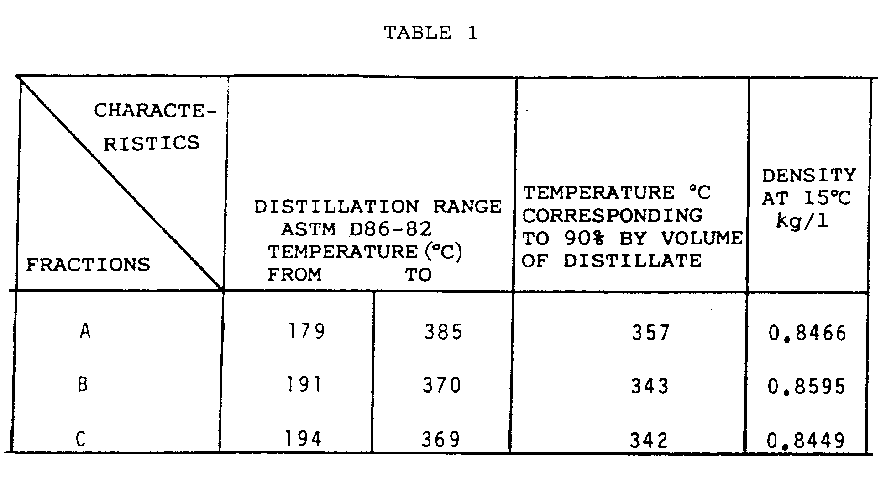

The tests relate to the following properties of the hydrocarbon fractions:
- C.P. (°C) =: Cloud Point according to ASTM-D 2500-81
- C.F.P.P. (°C) =: Cold Filter Plugging Point according to IP 309/83
- P.P. (°C) =: Pour Point according to ASTM-D 97-66.

### EXAMPLE 1

### Preparation of 3,5,10,12,17,19,24,26-octa-octadecanoyloxy-r-1,c-8,c-15,c-22-tetrahenaicosan-[l₄]metacyclophane.

### Part A:

1.65 g (15 mM) of resorcinol are dissolved, under an argon atmosphere, in 6 ml of ethanol and 3 ml of aqueous HCl at 37%. The solution is cooled down with an ice bath to 5°C and 4.87 g (15 mM) of 1-docosanal, dissolved in 70 ml of ethanol, are added dropwise. The mixture is then heated at reflux for 8 hours and then is cooled to room temperature. The precipitate obtained is filtered, washed with water until neutral, dried under vacum (25°C/1x10⁻³ mm Hg) and finally crystallized from ethanol. 4.31 g of pure tetramer are obtained (69% yield). ¹H-NMR (CDCl₃):δ=0.90 (t, 12H, CH₃); 1.28 (bs, 152H, (CH₂)₁₉); 2.31 (bm, 8H, CH-CH₂); 4.33 (t, 4H, CH); 6.14 (s, 4H, Ar-Hₐ); 7.23 (s, 4H, Ar-H_{b}); 9.20 (s, 4H, OHₐ); 9.50 (s, 4H, OH_{b}).

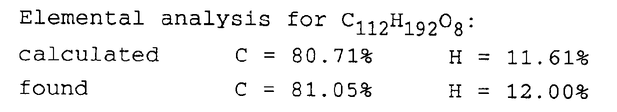

### Part B:

0.420 g (0.25 mM) of the tetramer obtained in Part A and 3.02 g (10 mM) of stearoyl chloride are heated for 8 hours at 150°C under magnetic stirring.

At the end of the reaction the excess chloride is distilled off under vacuum (140°C/1x10⁻² mm Hg).

The residue is dissolved in methylene chloride and the solution obtained is extracted with NaOH (0.2N), then washed with water until neutral and finally dried over anhydrous sodium sulphate.

The solvent is evaporated and the residue is purified on a silica gel column with hexane/methylene chloride 65/35 as eluent. 0.62 g of pure product are obtained (65% yield).

Mass (DCI-): M⁻-CO-C₁₇H₃₅ = 3527 (s ¹³C).
¹H-NMR (CDCl₃): δ= 0.86[m, 36H, (CH₂)ₙ-CH₃)]; 1.26 {(bs, 376H, 2x [(CH₂)₁₄] + (CH₂)₁₉}; 1.45 (m, 8H, CH-CH₂); 1.64 (m, 16H, CO-CH₂-CH₂); 2.25 (m, 8H, CO-CH₂); 2.56 (m, 8H, CO-CH₂'); 4.09 (t, 4H, CH); 5.99 (s, 2H, Ar-Hₐ); 6.70 (s, 2H, Ar-H_{b}); 6.73 (s, 2H, Ar-H_{b}'); 7.21 (s, 2H, Ar-Hₐ').

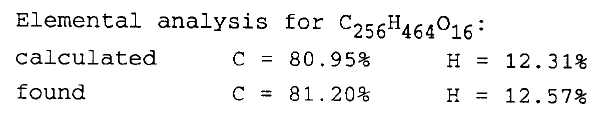

The product thus obtained, dissolved in SHELLSOL ® AB (10% concentration), was added to the hydrocarbon fractions A and B specified in Table 1 in the quantities shown below.

The following Table 2 gives the final results (in °C).

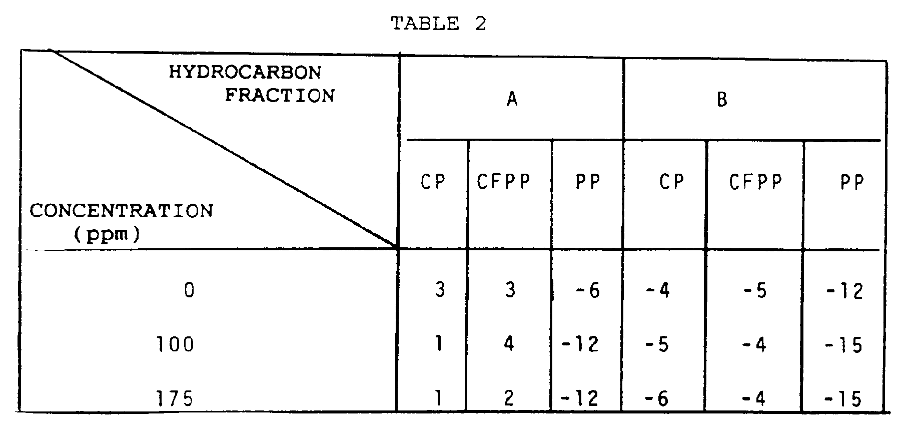

### EXAMPLE 2

### Preparation of 3,4,5,10,11,12,17,18,19,24,25,26-dodeca-hexadecyloxy-r-1,c-8,c-15,c-22-tetramethyl-[l₄]metacyclophane.

0.609 g (1 mM) of tetramer obtained as described in example 1, Part A, with the exception that the 1-docosanal was substituted by acetaldehyde and resorcinol was replaced by pyrrogallol, are dissolved in 50 ml of anhydrous DMF.

12.21 g (40 mM) of 1-bromohexadecane and 7.19 g (54 mM) of potassium carbonate are added to the solution. The mixture is heated to 80°C for 48 hrs and thereafter the mixture is diluted with water and extracted with diethyl ether. The organic phase is then separated, washed with water until neutral and dried over anhydrous sodium sulphate. The solvent is evaporated and the residue is purified on a silica gel column (two successive purifications are carried out, the first using hexane/methylene chloride 7/3 as the eluent and the second using hexane/methylene chloride 75/25). 1.45 g of pure product are obtained (44% yield).
Mass (DCI⁺): M⁺ = 3298 (2 ¹³C).
¹H-MMR (CDCl₃): δ= 0.86 [t, 36H, (CH₂)ₙ-CH₃]; 1.24 [bs, 312H, (CH₂)₁₃]; 1.40 (m, 12H, O-CH₂-CH₂); 1.51 (d, 12H, J=7.4 Hz, CH-CH₃); 1.61 (m, 12H,O-CH₂-CH₂'); 3.12 (m, 4H, O-CH₂); 3.61 (m, 8H, 0-CH₂'); 4.07 (m, 12H, O-CH₂''); 4.58 (q, 4H, J=7.4 Hz, CH); 5.67 (s, 2H, Ar-H); 6.97 (s, 2H, Ar-H').

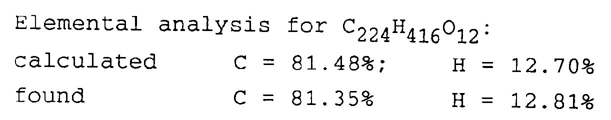

The product thus obtained, dissolved in SHELLSOL ® AB (10%), was added to the hydrocarbon fractions specified in Table 1.

The following Table 3 shows the final results.

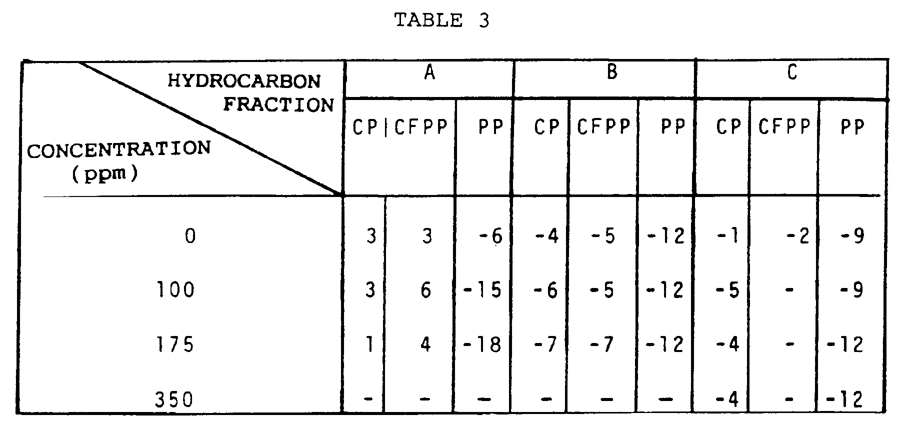

### EXAMPLE 3

### Preparation of 3,4,5,10,11,12,17,18,19,24,25,26-dodeca-octadecanoyloxy-r-1,c-8,c-15,c-22-tetraundecyl-[l₄]metacyclophane.

### Part A:

3.03 g (24 mM) of pyrogallol are dissolved, under an argon atmosphere, in 20 ml of ethanol and 3 ml of aqueous HCl (37%). The solution is cooled in an ice and salt bath to 0°C and 4.42 g (24 mM) of lauryl aldehyde are added dropwise. The solution is kept at room temperature under stirring for 24 hours and is then heated at reflux for 4 hours whereafter it is cooled slowly to room temperature. The precipitate obtained is filtered, washed with water until neutral and crystallized from ethanol. The solid product thus obtained is filtered and dried under vacuum (25°C/1.10⁻⁵ mm Hg): 1.60 g of product containing two ethanol molecules per tetramer molecule, are obtained.
¹H-NMR (acetone-d₆): δ= 0.88 (t, 12H, CH₃); 1.31 [bs, 72H, (CH₂)₉]; 2.28 (m, 8H, CH-CH₂); 4.35 (t, 4H, CH); 7.09 (s, 4H, Ar-H); 9.20 (bs, 4H, OHₐ); 9.45 (bs, 8H, OH_{b}).

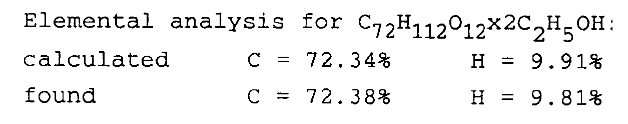

### Part B:

Following the procedure described in Example 1, Part B, by using octadecanoyl chloride, the title compound was obtained with a 60% yield, after purification on a silica gel column, using hexane/methylene chloride (7/3) as eluent. Mass (DCI⁻): M⁻ = 4363 (3 ¹³C)
¹H-NMR (CDCl₃): δ= 0.86 (m, 48H, (CH₂)ₙ-CH₃ ); 1.30 {bs, 408H, 3x(CH₂)₁₄-(CH₂)₉}; 1.45 (m, 8H, CH-CH₂); 1.61 (m, 24H, CO-CH₂-CH₂); 2.25 (m, 12H, CO-CH₂); 2.47 (m, 12H, CO-CH₂'); 4.08 (t, 4H, CH); 6.02 (s, 2H, Ar-H); 7.21 (s, 2H, Ar-H').

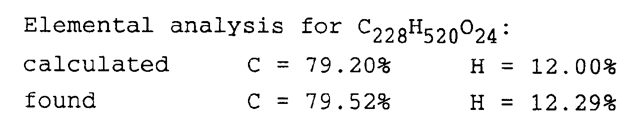

The product thus obtained, dissolved in SHELLSOL ® AB (10%), was added to the hydrocarbon fractions specified in Table 1.

The following Table 4 shows the final results.

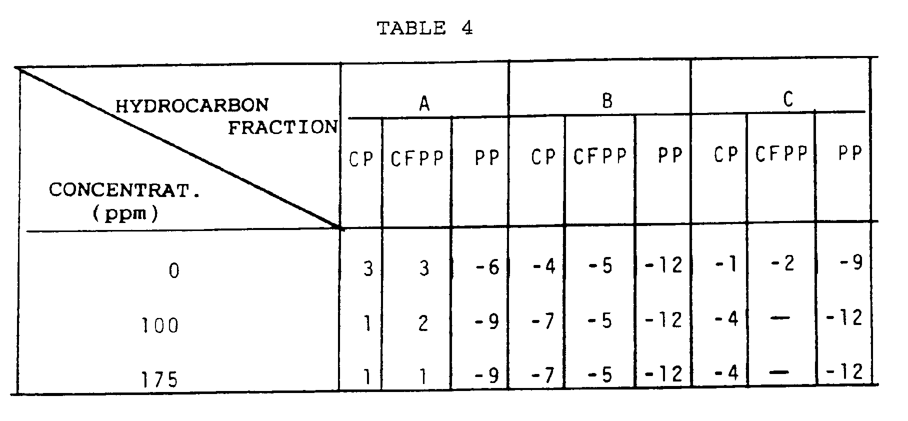

### EXAMPLE 4

The same procedure is used as in example 2 with the exception that the tetramer obtained in the first part is reacted with an equimolar mixture of the following acid chlorides: lauroyl chloride, palmitoyl chloride, stearoyl chloride, docosanoyl chloride. A mixture of substituted products having a molecular weight of from 2792 to 4472 is obtained after purification on a silica gel column (eluent methylene chloride/hexane 8/2).

The mixture of products thus obtained, dissolved in SHELLSOL® AB (10%), was added to the hydrocarbon fractions A and B of Table 1.
The following Table 5 shows the final results.

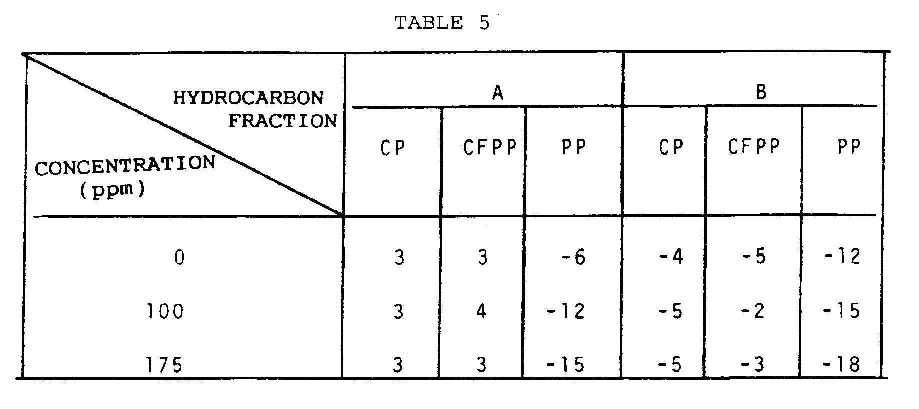

### EXAMPLE 5

### Preparation of 4,11,18,25-tetramethyl-3,5,10,12,17,19,24,26-octa-dodecanoyloxy-[l₄]metacyclophane

### Part A:

37.3 g (300 mM) of 2,6-dihydroxytoluene are dissolved, under an argon atmosphere, in 120 ml of degassed ethanol and 30 ml of 37% aqueous HCl. The solution is cooled to 5°C in an ice-bath and 25.7 ml (326 mM) of 35% formaldehyde in water are added dropwise over two hours. The solution is left at room temperature for 48 hours and is then heated to reflux for 6 hours. After cooling, a precipitate is obtained which is then filtered. On adding water to the solution a further precipitate is obtained. The two precipitates are combined, washed with water until neutral and crystallized from acetone. The precipitate thus obtained is filtered and dried under vacuum (25°C/1x10⁻⁵ mm Hg). 25.0 g of product are thus obtained (61% yield).
¹H-NMR (DMSO-d₆): δ= 2.08 (s, 12H, CH₃); 3.67 (s, 8H, CH₂); 6.82 (s, 4H, Ar-H); 8.68 (s, 8H, OH).

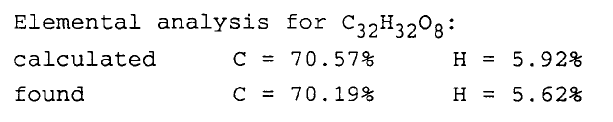

### Part B:

Following the procedure described in example 1, Part B, by using dodecanoyl chloride, the title compound was obtained with a 75% yield, after two crystallizations from acetone. Mass (DCI⁺): MH⁺ = 2002 (1 ¹³C)
¹H-NMR (CDCl₃): δ= 0.89 (t, 24H, (CH₂)ₙ-CH₃); 1.25 [bs, 128H, (CH₂)₈]; 1.68 (m, 16H, CO-CH₂-CH₂); 1.89 (2, 12H, Ar-CH₃); 2.51 (t, 16H, CO-CH₂); 3.50 (s, 8H, Ar-CH₂-Ar); 6.38 (s, 4H, Ar-H).

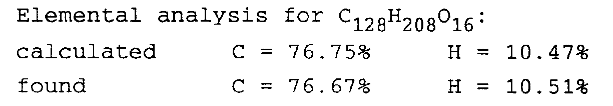

The product thus obtained, dissolved in SHELLSOL ® AB (10%) was added to hydrocarbon fraction B shown in Table 1. The following Table 6 shows the final results.

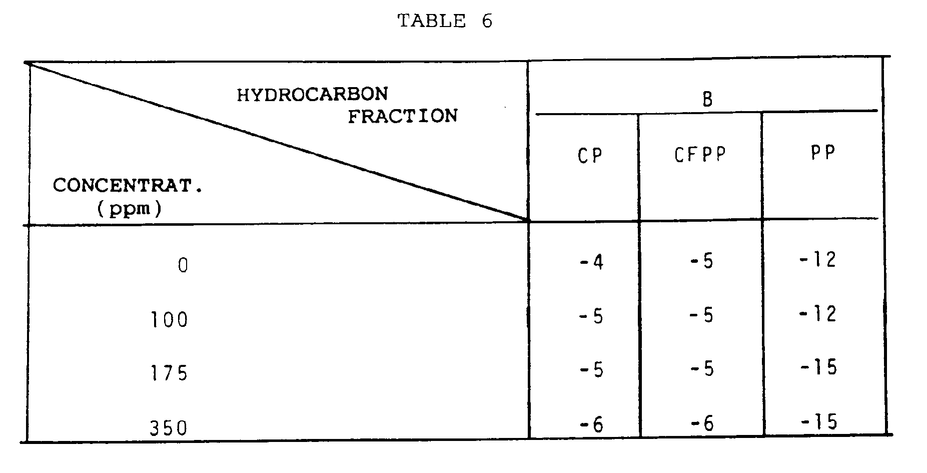

### EXAMPLE 6

### Preparation of 4,11,18,25-tetramethyl-3,5,10,12,17,19,24,26-octa-octadecanoyloxy-[l₄] metacyclophane

The same procedure is used as in example 1, Part B, by reacting stearoyl chloride with the tetramer obtained in example 5, Part A. The title compound was obtained with a 60% yield after purification on a silica gel column using methylene chloride/hexane 9/1 as eluent.
Mass (DCI⁺): M⁺ = 2673 (1 ¹³C)
¹H-NMR (CDCl₃): δ= 0.86 (t, 24H, (CH₂)ₙ-CH₃); 1.24 [bs, 224H, (CH₂)₁₄]; 1.66 (m, 16H, CO-CH₂-CH₂); 1.88 (s, 12H, Ar-CH₃); 2.47 (t, 16H, CO-CH₂); 3.49 (s, 8H, Ar-CH₂-Ar); 6.36 (s, 4H, Ar-H).

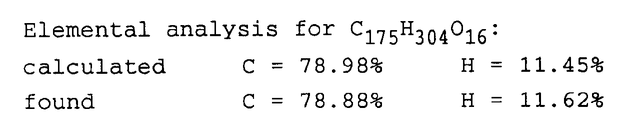

The product thus obtained, dissolved in SHELLSOL ® AB (10%), was added to the hydrocarbon fractions A and B of Table 1. The following Table 7 shows the final results.

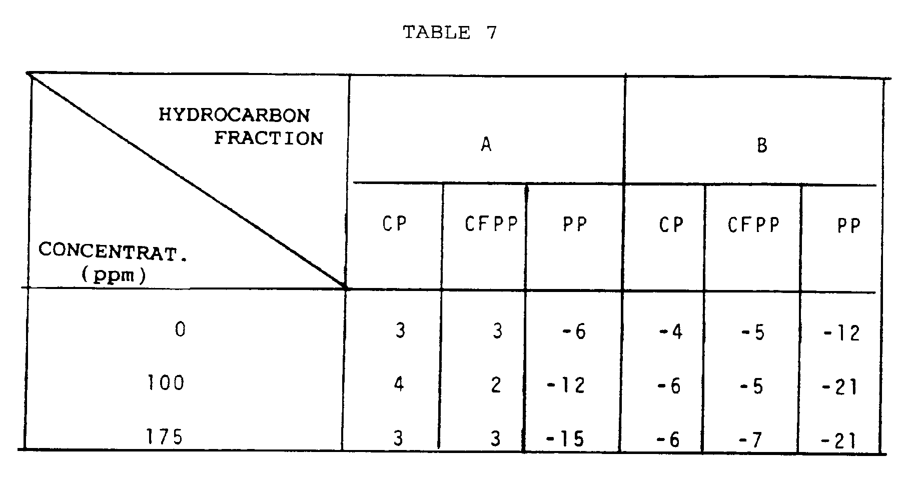

### EXAMPLE 7

### Preparation of 3,5,10,12,17,19,24,26-octa-acetyloxy-r-1,c-8,c-15,c-22-tetraheptadecyl-[l₄] metacyclophane.

### Part A:

The same procedure is used as in example 1, Part A, using stearic aldehyde instead of 1-docosanal; yield: 82%.
¹H-NMR (CD₃OD, 84°C); δ= 0.91 [t, 12H, (CH₂)ₙ-CH₃)]; 1.36 [bs, 120H, (CH₂)₁₅]; 2.16 (m, 8H, CH-CH₂): 4.29 (t, 4H, CH); 6.21 (s, 4H, Ar-Hₐ); 7.16 (s, 4H, Ar-H_{b}).

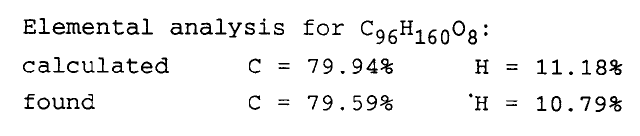

### Part B:

1.50 g (1.04 mM) of the product of Part A are suspended in 10 ml of acetic anhydride containing two drops of pyridine. The mixture is heated to reflux under stirring for one hour. The solid precipitate thus obtained, after cooling, is filtered, washed with water until neutral and crystallized from ethanol. The precipitate is dried under vacuum (1.10⁻³ mm Hg). Thus 1.38 g of pure product are obtained (75% yield).
Mass (DCI⁺): MH⁺ = 1778 (1 ¹³C).
¹H-NMR (CDCl₃): δ= 0.89 [t, 12H, (CH₂)ₙ-CH₃)]; 1.29 [bs, 120H, (CH₂)₁₅]; 1.84 (m, 8H, CH-CH₂); 2.13 (bs, 24H,CO-CH₃); 4.12 (t, 4H, CH); 6.10 (s, 4H, Ar-Hₐ); 6.89 (s, 4H, Ar-H_{b}).

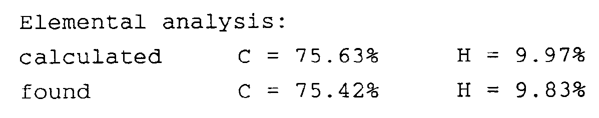

The product thus obtained, dissolved in SHELLSOL ® AB (10%), was added to hydrocarbon fractions A and B shown in Table 1. Table 8 below shows the final results.

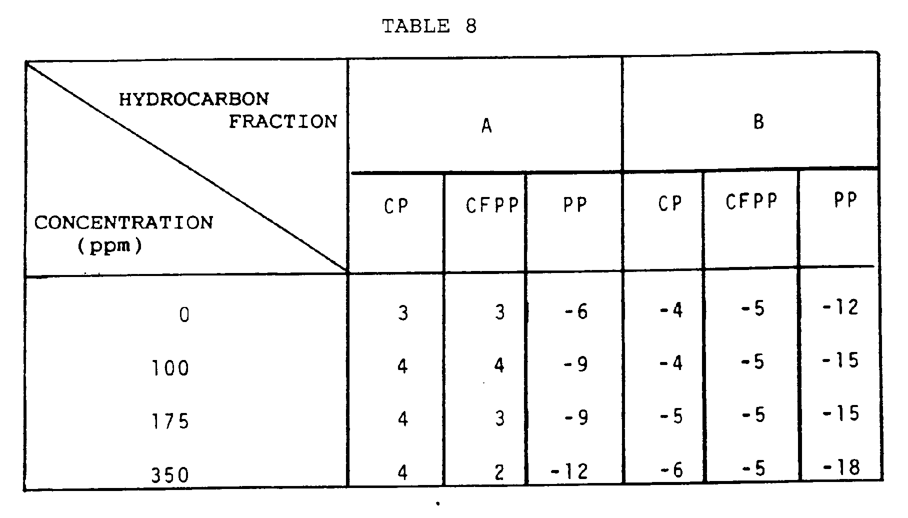

### EXAMPLE 8

### Preparation of 3,5,10,12,17,19,24,26-octa-octadecanoyloxy-r-1,c-8,c-15,c-22-tetramethyl-[l₄] metacyclophane.

0.545 g (1 mM) of tetramer obtained in Part A of example 1, with the exception that acetaldehyde is used instead of 1-docosanal, and 19.5 g of stearoyl chloride are heated to about 180°C for 6 hours under stirring. At the end of the reaction, the excess chloride is distilled off under vacuum (140°C/1x10⁻² mm Hg).

The residue is dissolved in methylene chloride and the solution obtained is first mixed and shaken with NaOH (0.2 N), then washed with water until neutral and finally dried over anhydrous sodium sulphate. The solvent is evaporated and the residue is passed, for two hours, over a silica gel column using methylene chloride/hexane 7/3 and methylene chloride/hexane 6/4 as eluents. 1.66 g of pure product are thus obtained (62% yield).
Mass (DCI⁺): M⁺ = 2673 (1 ¹³C).
¹H-NMR (CDCl₃): δ= 0.87 [t, 24H, (CH₂)ₙ-CH₃)]; 1.28 [bs, 224H, (CH₂)₁₄]; 1.43 (d, 12H, J = 7.5 Hz, CH-CH₃); 1.51 (m, 8H, CO-CH₂-CH₂); 1.75 (m, 8H, CO-CH₂-CH₂'); 2.22 (m, 8H, CO-CH₂); 2.57 (m, 8H, CO-CH₂'); 4.22 (q, 4H, J = 7.5 Hz, CH); 5.91 (s, 2H, Ar-Hₐ); 6.72 (s, 2H, Ar-H_{b}); 6.88 (s, 2H, Ar-H_{b}'); 7.38 (s, 2H, Ar-Hₐ').

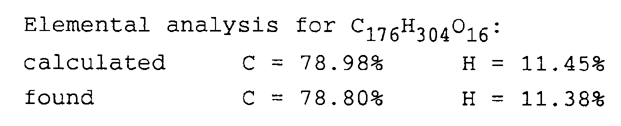

175 ppm of the product thus obtained were added, after 10% dilution with SHELLSOL ® AB, to the A and B hydrocarbon fractions of Table 1 and the Pour Point was decreased respectively from -6 to -18°C and from -12 to -21°C.
350 ppm of the same product, added to fraction C of Table 1, reduced the Cloud Point thereof from -1 to -4°C.

### EXAMPLE 9

### Preparation of 3,4,5,10,11,12,17,18,19,24,25,26-dodeca-octadecanoyloxy-r-1,c-8,c-15,c-22-tetramethyl-[l₄]metacyclophane.

0.609 g (1 mM) of tetramer obtained in Part A of example 2 and 12.12 g (40 mM) of stearoyl chloride are reacted, using the same procedure as in example 8. In this case the product is purified by two crystallizations from methylene chloride/diethyl ether (53% yield).
Mass (DCI⁻) : M⁻ = 3802 (2 ¹³C)
¹H-NMR (CDCl₃): δ= 0.89 [t, 36H, (CH₂)ₙ-CH₃)]; 1.30 [bs, 336H, (CH₂)₁₄]; 1.46 (d, 12H, J = 7.4 Hs, CH-CH₃); 1.70 (m, 24H, CO-CH₂-CH₂); 2.30 (m, 12H, CO-CH₂); 2.51 (m, 12H, CO-CH₂'); 4.23 (q, 4H, J = 7.4 Hz, CH); 6.03 (s, 2H, Ar-H); 7.30 (s, 2H, Ar-H').

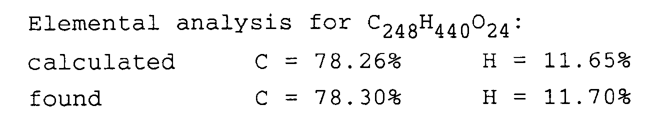

175 ppm of the product thus obtained were added, after 10% dilution with SHELLSOL ® AB, to hydrocarbon fraction A of Table 1 and the Pour Point thereof was decreased from 3 to 0°C.

## Claims (Claims for the following Contracting State(s): AT, BE, DE, DK, FR, GB, GR, NL, SE)

1. Compositions of refinery hydrocarbons, comprising at least one anti-cloud additive of general formula (I): 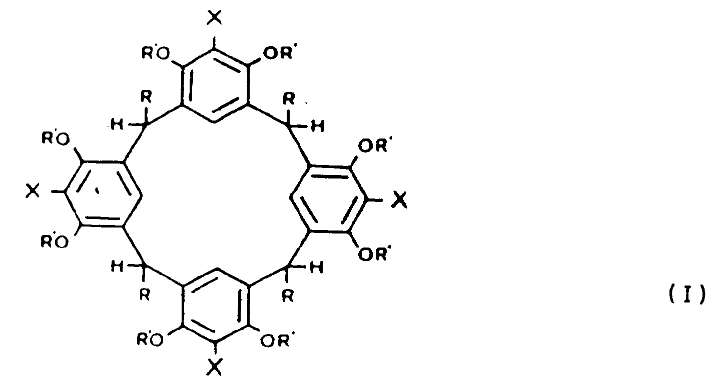 wherein the groups R are selected from hydrogen, C₁-C₃₀ alkyl, iso-alkyl, cyclo-alkyl, aryl and alkyl-aryl; the groups R' are independently selected from C₁-C₃₀ alkyl, iso-alkyl, cyclo-alkyl, aryl and alkyl-aryl or at least one of said groups has one of the general formulae (II) to (IV):
-COR₁ (II) 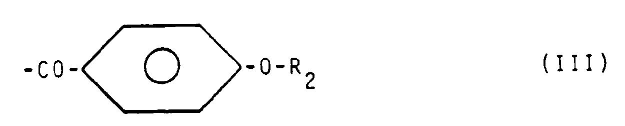 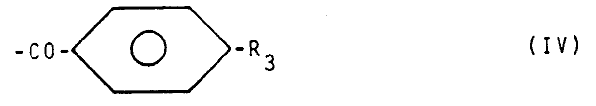
where R₁, R₂, R₃, the same or different from each other, represent C₁-C₃₀ alkyl, iso-alkyl, cyclo-alkyl, aryl or alkyl-aryl; and X is hydrogen, lower alkyl or -OR', R' having the meanings given above.

2. Compositions according to claim 1 wherein X in general formula (I) is selected from hydrogen, C₈-C₂₀ alkoxy and C₁-C₄ alkyl groups, particularly from hydrogen, methyl and C₁₀-C₁₈ alkoxy.

3. Compositions according to any one of the preceding claims wherein R in general formula (I) is selected from hydrogen and C₁-C₂₀ alkyl, particularly from hydrogen, methyl and ethyl.

4. Compositions according to any one of the preceding claims wherein R' is selected from C₁-C₂₀ alkyl and groups of general formula (II) wherein R₁ is C₁-C₂₀ alkyl, particularly C₉-C₂₀ alkyl.

5. Compositions according to any one of claims 1 to 4, in which the additives of general formula (I) have been synthesized by reaction of resorcin, optionally substituted in position 2, with an aldehyde and subsequent esterification of the resulting product with a halide of formula R'-Y, where R' has the meanings given in claim 1 and Y is halogen, or with an acid anhydride.

6. Compositions according to any one of claims 1 to 5, in which the additives of general formula (I) have been added to the hydrocarbon fractions as such or dissolved in a solvent.

7. Compositions according to any one of the preceding claims, in which the additives have been added to the hydrocarbon fractions in amounts of at least 0.001% by weight, preferably of from 0.001 to 1 and particularly of from 0.005 to 0.15% by weight.

8. Compositions according to any one of the preceding claims showing a decrease of the Cloud Point of up to 3°C and a decrease of the Pour Point of at least 10°C, as compared to the corresponding compositions without additive.

9. Process for the preparation of hydrocarbon compositions according to any one of the preceding claims, comprising the addition of at least one additive of general formula (I) to a Middle Distillate in an amount of at least 0.001% by weight.

10. Substituted [l₄]-metacyclophanes of general formula (V): 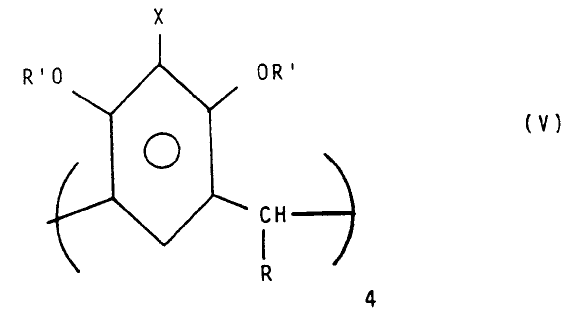 wherein X is
i) hydrogen, and at the same time R is a linear C₄-C₃₀ alkyl or a C₃-C₃₀ iso-alkyl, cycloalkyl, aryl or alkyl-aryl group and the radicals R',alike or different from each other, represent groups of general formulae (II) to (IV):
-COR₁ (II) 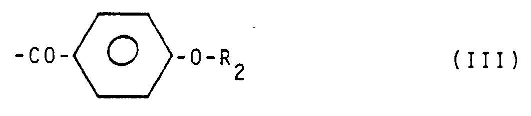 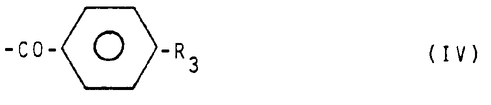
where R₁, R₂ and R₃, alike or different from each other, are selected from C₁-C₃₀ alkyl, iso-alkyl, cyclo-alkyl, aryl and alkyl-aryl groups;
ii) a lower alkyl group and at the same time R is hydrogen or C₁-C₃₀ alkyl, iso-alkyl, cyclo-alkyl, aryl or alkyl-aryl and R' represents groups of general formulae (II), (III) and (IV), where R₁, R₂ and R₃, alike or different from each other, are C₁-C₃₀ alkyl, iso-alkyl, cyclo-alkyl, aryl or alkyl-aryl groups;
iii) a group OR', and at the same time R is C₃-C₃₀ alkyl, iso-alkyl, cyclo-alkyl, aryl or alkyl-aryl and R' represents groups of general formulae (II), (III) and (IV) as defined above;
iv) a group OR'' and at the same time R is C₁-C₃₀ alkyl, iso-alkyl, cyclo-alkyl, aryl or alkyl-aryl and R' and R'', both the same, are C₁-C₃₀ alkyl, iso-alkyl, cyclo-alkyl, aryl or alkyl-aryl groups.

## Claims (Claims for the following Contracting State(s): ES)

1. Compositions of refinery hydrocarbons, comprising at least one anti-cloud additive of general formula (I): 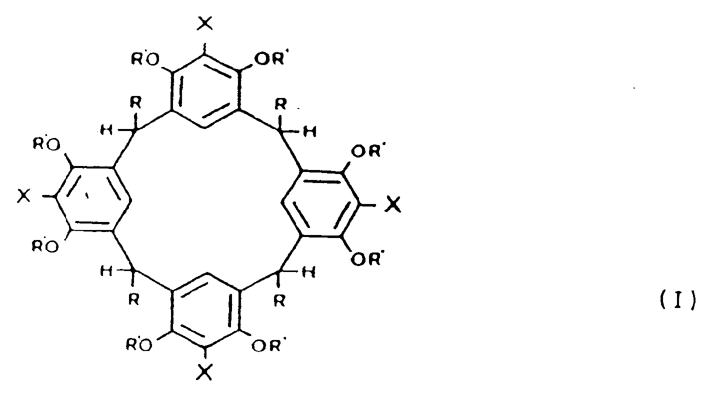 wherein the groups R are selected from hydrogen, C₁-C₃₀ alkyl, iso-alkyl, cyclo-alkyl, aryl and alkyl-aryl; the groups R' are independently selected from C₁-C₃₀ alkyl, iso-alkyl, cyclo-alkyl, aryl and alkyl-aryl or at least one of said groups has one of the general formulae (II) to (IV):
-COR₁ (II) 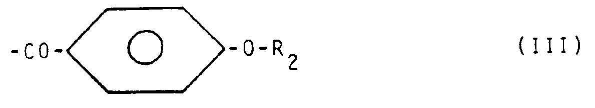 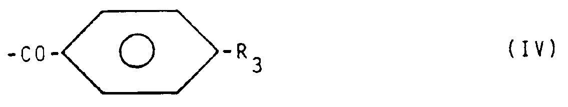
where R₁, R₂, R₃, the same or different from each other, represent C₁-C₃₀ alkyl, iso-alkyl, cyclo-alkyl, aryl or alkyl-aryl; and X is hydrogen, lower alkyl or -OR', R' having the meanings given above.

2. Compositions according to claim 1 wherein X in general formula (I) is selected from hydrogen, C₈-C₂₀ alkoxy and C₁-C₄ alkyl groups, particularly from hydrogen, methyl and C₁₀-C₁₈ alkoxy.

3. Compositions according to any one of the preceding claims wherein R in general formula (I) is selected from hydrogen and C₁-C₂₀ alkyl, particularly from hydrogen, methyl and ethyl.

4. Compositions according to any one of the preceding claims wherein R' is selected from C₁-C₂₀ alkyl and groups of general formula (II) wherein R₁ is C₁-C₂₀ alkyl, particularly C₉-C₂₀ alkyl.

5. Compositions according to any one of claims 1 to 4, in which the additives of general formula (I) have been synthesized by reaction of resorcin, optionally substituted in position 2, with an aldehyde and subsequent esterification of the resulting product with a halide of formula R'-Y, where R' has the meanings given in claim 1 and Y is halogen, or with an acid anhydride.

6. Compositions according to any one of claims 1 to 5, in which the additives of general formula (I) have been added to the hydrocarbon fractions as such or dissolved in a solvent.

7. Compositions according to any one of the preceding claims, in which the additives have been added to the hydrocarbon fractions in amounts of at least 0.001% by weight, preferably of from 0.001 to 1 and particularly of from 0.005 to 0.15% by weight.

8. Compositions according to any one of the preceding claims showing a decrease of the Cloud Point of up to 3°C and a decrease of the Pour Point of at least 10°C, as compared to the corresponding compositions without additive.

9. Process for the preparation of hydrocarbon compositions according to any one of the preceding claims, comprising the addition of at least one additive of general formula (I) to a Middle Distillate in an amount of at least 0.001% by weight.

## Patentansprüche (Patentansprüche für folgende(n) Vertragsstaat(en): AT, BE, DE, DK, FR, GB, GR, NL, SE)

1. Raffineriekohlenwasserstoffzusammensetzungen, die mindestens einen Antitrübungszusatz der allgemeinen Formel (I) 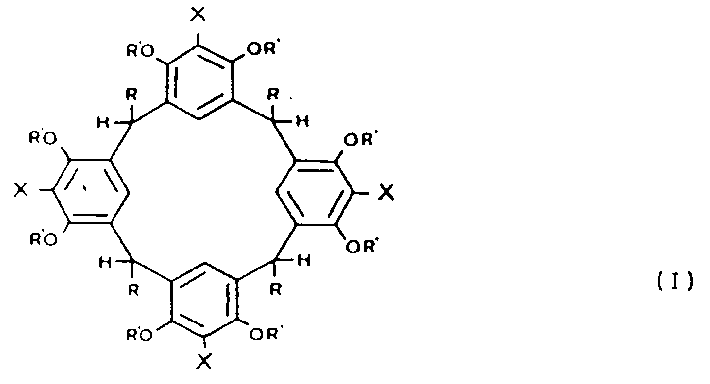 enthalten, in der die Gruppen R ausgewählt sind aus Wasserstoff, C₁-C₃₀-Alkyl, Isoalkyl, Cycloalkyl, Aryl und Alkylaryl, die Gruppen R' unabhängig ausgewählt sind aus C₁-C₃₀-Alkyl, Isoalkyl, Cycloalkyl, Aryl und Alkylaryl oder in der mindestens eine dieser Gruppen eine Gruppe der allgemeinen Formeln (II) bis (IV) aufweist:
-COR₁ (II) 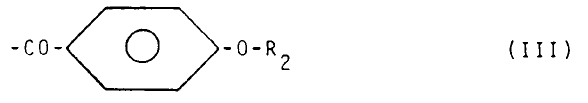 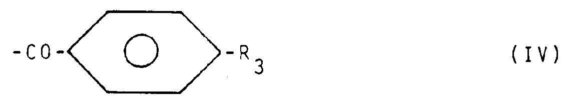
in denen R₁, R₂, R₃, die gleich oder voneinander verschieden sein können, C₁-C₃₀-Alkyl, Isoalkyl, Cycloalkyl, Aryl oder Alkylaryl bedeuten, und worin X Wasserstoff, Niederalkyl oder -OR' ist, wobei R' die obigen Bedeutungen hat.

2. Zusammensetzung nach Anspruch 1, worin X in der allgemeinen Formel (I) ausgewählt ist aus Wasserstoff, C₈-C₂₀-Alkoxy- und C₁-C₄-Alkylgruppen, insbesondere aus Wasserstoff, Methyl und C₁₀-C₁₈-Alkoxy.

3. Zusammensetzung nach irgendeinem der vorhergehenden Ansprüche, worin R in der allgemeinen Formel (I) ausgewählt ist aus Wasserstoff und C₁-C₂₀-Alkyl, insbesondere aus Wasserstoff, Methyl und Ethyl.

4. Zusammensetzung nach irgendeinem der vorhergehenden Ansprüche, worin R' ausgewählt ist aus C₁-C₂₀-Alkyl und Gruppen der allgemeinen Formel (II), worin R₁ C₁-C₂₀-Alkyl, insbesondere C₉-C₂₀-Alkyl, ist.

5. Zusammensetzung nach irgendeinem der Ansprüche 1 bis 4, in der die Additive der allgemeinen Formel (I) hergestellt wurden durch Reaktion von, gegebenenfalls in 2-Stellung substituiertem, Resorcin mit einem Aldehyd und anschließende Veresterung des resultierenden Produkts mit einem Halogenid der Formel R'-Y, in der R' die in Anspruch 1 angegebenen Bedeutungen hat und Y Halogen ist, oder mit einem Säureanhydrid.

6. Zusammensetzung nach irgendeinem der Ansprüche 1 bis 5, in der die Additive der allgemeinen Formel (I) als solche oder in einem Lösemittel gelöst der Kohlenwasserstofffraktion zugesetzt wurden.

7. Zusammensetzung nach irgendeinem der vorhergehenden Ansprüche, in der die Additive der Kohlenwasserstofffraktion in Mengen von mindestens 0.001 Gew.-%, vorzugsweise von 0.001 bis 1 Gew.-% und insbesondere von 0.005 bis 0.15 Gew.-%, zugesetzt wurden.

8. Zusammensetzung nach irgendeinem der vorhergehenden Ansprüche, die eine Erhöhung des Trübungspunktes von bis zu 3 °C und eine Erhöhung des Stockpunktes von mindestens 10 °C zeigen, verglichen mit den entsprechenden Zusammensetzungen ohne Zusatz.

9. Verfahren zur Herstellung der Kohlenwasserstoffzusammensetzungen nach irgendeinem der vorhergehenden Ansprüche, das die Zugabe von mindestens einem Zusatz der allgemeinen Formel (I) zu einem Destillationsmittelschnitt in einer Menge von mindestens 0.001 Gew.-% umfaßt.

10. Substituierte [I₄] -Metacyclophane der allgemeinen Formel (V) 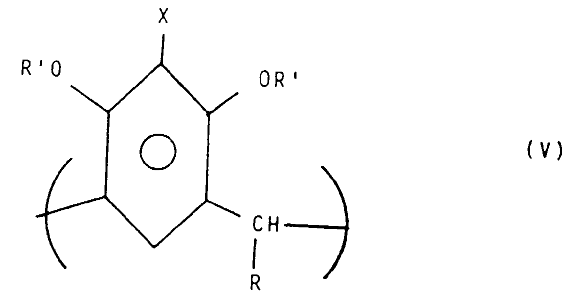 in der X bedeutet:
i) Wasserstoff, wobei gleichzeitig R eine lineare C₄-C₃₀-Alkyl- oder C₃-C₃₀-Isoalkyl-, Cycloalkyl-, Aryl- oder Alkylarylgruppe ist und die Reste R', gleich oder voneinander verschieden, Gruppen der allgemeinen Formeln (II) bis (IV) bedeuten
-COR₁ (II) 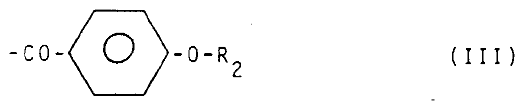 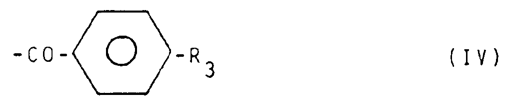
worin R₁, R₂ und R₃, gleich oder voneinander verschieden, ausgewählt sind aus C₁-C₃₀-Alkyl-, Isoalkyl-, Cycloalkyl-, Aryl- und Alkylarylgruppen,
ii) eine Niederalkylgruppe, wobei gleichzeitig R Wasserstoff oder eine C₁-C₃₀-Alkyl-, Isoalkyl, Cycloalkyl-, Aryl- oder Alkylarylgruppe ist und R' Gruppen der allgemeinen Formeln (II), (III) und (IV) bedeutet, worin R₁, R₂ und R₃, gleich oder voneinander verschieden, C₁-C₃₀-Alkyl-, Isoalkyl-, Cycloalkyl-, Aryl- und Alkylarylgruppen sind,
iii) eine Gruppe OR', wobei gleichzeitig R C₃-C₃₀-Alkyl, Isoalkyl, Cycloalkyl, Aryl oder Alkylaryl ist und R' Gruppen der wie oben definierten allgemeinen Formeln (II), (III) und (IV) bedeutet,
iv) eine Gruppe OR'', wobei gleichzeitig R C₁-C₃₀-Alkyl, Isoalkyl, Cycloalkyl, Aryl oder Alkylaryl ist und R' und R'', beide gleich, C₁-C₃₀-Alkyl-, Isoalkyl-, Cycloalkyl-, Aryl- und Alkylarylgruppen sind.

## Patentansprüche (Patentansprüche für folgende(n) Vertragsstaat(en): ES)

1. Raffineriekohlenwasserstoffzusammensetzungen, die mindestens einen Antitrübungszusatz der allgemeinen Formel (I) 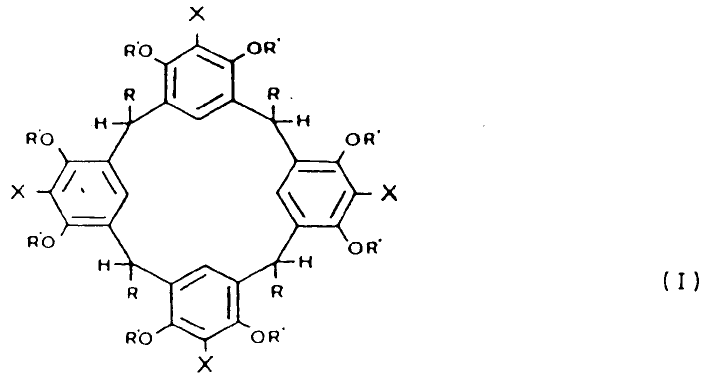 enthalten, in der die Gruppen R ausgewählt sind aus Wasserstoff, C₁-C₃₀-Alkyl, Isoalkyl, Cycloalkyl, Aryl und Alkylaryl, die Gruppen R' unabhängig ausgewählt sind aus C₁-C₃₀-Alkyl, Isoalkyl, Cycloalkyl, Aryl und Alkylaryl oder in der mindestens eine dieser Gruppen eine Gruppe der allgemeinen Formeln (II) bis (IV) aufweist:
-COR₁ (II) 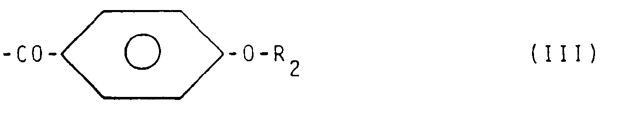 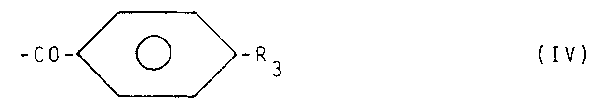
in denen R₁, R₂, R₃, die gleich oder voneinander verschieden sein können, C₁-C₃₀-Alkyl, Isoalkyl, Cycloalkyl, Aryl oder Alkylaryl bedeuten, und worin X Wasserstoff, Niederalkyl oder -OR' ist, wobei R' die obigen Bedeutungen hat.

2. Zusammensetzung nach Anspruch 1, worin X in der allgemeinen Formel (I) ausgewählt ist aus Wasserstoff, C₈-C₂₀-Alkoxy- und C₁-C₄-Alkylgruppen, insbesondere aus Wasserstoff, Methyl und C₁₀-C₁₈-Alkoxy.

3. Zusammensetzung nach irgendeinem der vorhergehenden Ansprüche, worin R in der allgemeinen Formel (I) ausgewählt ist aus Wasserstoff und C₁-C₂₀-Alkyl, insbesondere aus Wasserstoff, Methyl und Ethyl.

4. Zusammensetzung nach irgendeinem der vorhergehenden Ansprüche, worin R' ausgewählt ist aus C₁-C₂₀-Alkyl und Gruppen der allgemeinen Formel (II), worin R₁ C₁-C₂₀-Alkyl, insbesondere C₉-C₂₀-Alkyl, ist.

5. Zusammensetzung nach irgendeinem der Ansprüche 1 bis 4, in der die Additive der allgemeinen Formel (I) hergestellt wurden durch Reaktion von, gegebenenfalls in 2-Stellung substituiertem, Resorcin mit einem Aldehyd und anschließende Veresterung des resultierenden Produkts mit einem Halogenid der Formel R'-Y, in der R' die in Anspruch 1 angegebenen Bedeutungen hat und Y Halogen ist, oder mit einem Säureanhydrid.

6. Zusammensetzung nach irgendeinem der Ansprüche 1 bis 5, in der die Additive der allgemeinen Formel (I) als solche oder in einem Lösemittel gelöst der Kohlenwasserstofffraktion zugesetzt wurden.

7. Zusammensetzung nach irgendeinem der vorhergehenden Ansprüche, in der die Additive der Kohlenwasserstofffraktion in Mengen von mindestens 0.001 Gew.-%, vorzugsweise von 0.001 bis 1 Gew.-% und insbesondere von 0.005 bis 0.15 Gew.-%, zugesetzt wurden.

8. Zusammensetzung nach irgendeinem der vorhergehenden Ansprüche, die eine Erhöhung des Trübungspunktes von bis zu 3 °C und eine Erhöhung des Stockpunktes von mindestens 10 °C zeigen, verglichen mit den entsprechenden Zusammensetzungen ohne Zusatz.

9. Verfahren zur Herstellung der Kohlenwasserstoffzusammensetzungen nach irgendeinem der vorhergehenden Ansprüche, das die Zugabe von mindestens einem Zusatz der allgemeinen Formel (I) zu einem Destillationsmittelschnitt in einer Menge von mindestens 0.001 Gew.-% umfaßt.

## Revendications (Revendications pour l'(les) Etat(s) contractant(s) suivant(s): AT, BE, DE, DK, FR, GB, GR, NL, SE)

1. Compositions d'hydrocarbures de raffinerie, comprenant au moins un additif anti-trouble de formule générale (I) : 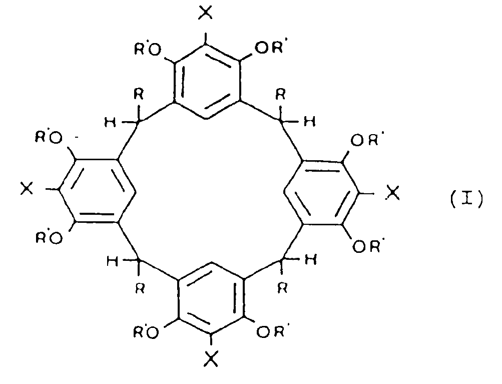 dans laquelle :
- les groupes R sont choisis parmi hydrogène, alkyle, isoalkyle, cyclo-alkyle, aryle et alkyl-aryle en C₁-C₃₀ ;
- les groupes R' sont choisis indépendamment parmi alkyle, iso-alkyle, cyclo-alkyle, aryle et alkyl-aryle en C₁-C₃₀,
- ou bien au moins l'un des groupes précités présente l'une des formules générales (II) à IV) :
-COR₁ (II) 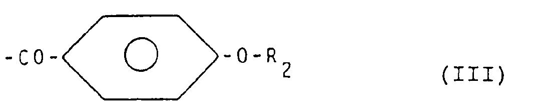 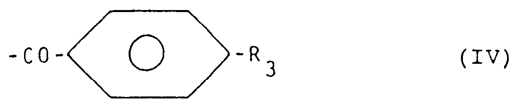
dans lesquelles R₁, R₂**,** R₃, identiques ou différents les uns des autres, représentent alkyle, iso-alkyle, cycloalkyle, aryle ou alkyl-aryle en C₁-C₃₀ ; et
- X représente hydrogène, alkyle inférieur ou -OR', R' ayant les significations données ci-dessus.

2. Compositions selon la revendication 1, dans lesquelles X, dans la formule générale (I), est choisi parmi hydrogène, les groupes alcoxy en C₈-C₂₀ et alkyle en C₁-C₄, en particulier, parmi hydrogène, méthyle et alcoxy en C₁₀-C₁₈.

3. Compositions selon l'une quelconque des revendications précédentes, dans lesquelles R, dans la formule générale (I), est choisi parmi hydrogène et alkyle en C₁-C₂₀, en particulier, parmi hydrogène, méthyle et éthyle.

4. Compositions selon l'une quelconque des revendications précédentes, dans lesquelles R' est choisi parmi alkyle en C₁-C₂₀ et les groupes de formule générale (II), dans laquelle R₁ représente alkyle en C₁-C₂₀, en particulier, alkyle en C₉-C₂₀.

5. Compositions selon l'une quelconque des revendications 1 à 4, dans lesquelles les additifs de formule générale (I) ont été synthétisés par réaction de la résorcine, facultativement substituée en position 2, avec un aldéhyde, et estérification ultérieure du produit résultant avec un halogénure de formule R'-Y, où R' a les significations données à la revendication 1 et Y représente halogène, ou avec un anhydride d'acide.

6. Compositions selon l'une quelconque des revendications 1 à 5, dans lesquelles les additifs de formule générale (I) ont été ajoutés aux fractions hydrocarbonées en tant que tels ou dissous dans un solvant.

7. Compositions selon l'une quelconque des revendications précédentes, dans lesquelles les additifs ont été ajoutés aux fractions hydrocarbonées dans des quantités d'au moins 0,001% en poids, de préférence, de 0,001 à 1, et en particulier, de 0,005 à 0,15% en poids.

8. Compositions selon l'une quelconque des revendications précédentes, montrant une diminution du Point de Trouble allant jusqu'à 3°C et une diminution du Point d'Ecoulement d'au moins 10°C, par comparaison avec les compositions correspondantes sans additif.

9. Procédé de fabrication des compositions d'hydrocarbures telles que définies à l'une quelconque des revendications précédentes, comprenant l'addition d'au moins un additif de formule générale (I) à un Distillat Moyen, dans une quantité d'au moins 0,001% en poids.

10. **[**l₄]-métacyclophanes substitués de formule générale (V) : 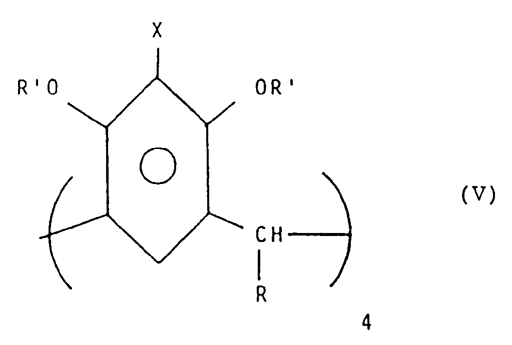 dans laquelle X représente :
(i) hydrogène, et, en même temps, R représente un groupe alkyle linéaire en C₄-C₃₀ ou un groupe iso-alkyle, cyclo-alkyle, aryle ou alkyl-aryle en C₃-C₃₀, et les radicaux R', identiques ou différents les uns des autres, représentent des groupes des formules générales (II) à (IV) :
-COR₁ (II) 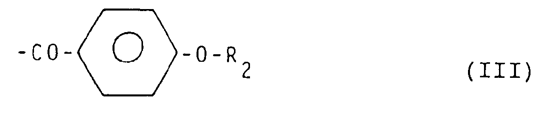 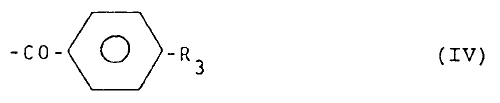
dans lesquelles R₁, R₂ et R₃, identiques ou différents les uns des autres, sont choisis parmi les groupes alkyle, iso-alkyle, cyclo-alkyle, aryle et alkyl-aryle en C₁-C₃₀ ;
(ii) un groupe alkyle inférieur et, en même temps, R représente hydrogène ou alkyle, iso-alkyle, cyclo-alkyle, aryle ou alkyl-aryle en C₁-C₃₀ et R' représente des groupes des formules générales (II), (III) et (IV), dans lesquelles R₁, R₂ et R₃, identiques ou différents les uns des autres, représentent des groupes alkyle, isoalkyle, cyclo-alkyle, aryle ou alkyl-aryle en C₁-C₃₀ ;
(iii) un groupe OR', et, en même temps, R représente alkyle, iso-alkyle, cyclo-alkyle, aryle ou alkyl-aryle en C₃-C₃₀ et R' représente des groupes des formules générales (II), (III) et (IV) telles que définies ci-dessus ;
(iv) un groupe OR'' et, en même temps, R représente alkyle, iso-alkyle, cyclo-alkyle, aryle ou alkyl-aryle en C₁-C₃₀ et R' et R'', tous deux identiques, représentent des groupes alkyle, iso-alkyle, cyclo-alkyle, aryle ou alkyl-aryle en C₁-C₃₀.

## Revendications (Revendications pour l'(les) Etat(s) contractant(s) suivant(s): ES)

1. Compositions d'hydrocarbures de raffinerie, comprenant au moins un additif anti-trouble de formule générale (I) : 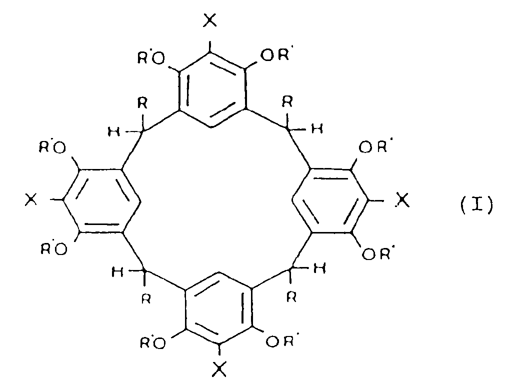 dans laquelle :
- les groupes R sont choisis parmi hydrogène, alkyle, isoalkyle, cyclo-alkyle, aryle et alkyl-aryle en C₁-C₃₀ ;
- les groupes R' sont choisis indépendamment parmi alkyle, iso-alkyle, cyclo-alkyle, aryle et alkyl-aryle en C₁-C₃₀,
- ou bien au moins l'un des groupes précités présente l'une des formules générales (II) à IV) :
-COR₁ (II) 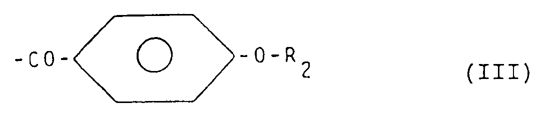 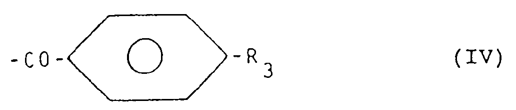
dans lesquelles R₁, R₂, R₃, identiques ou différents les uns des autres, représentent alkyle, iso-alkyle, cycloalkyle, aryle ou alkyl-aryle en C₁-C₃₀ ; et
- X représente hydrogène, alkyle inférieur ou -OR', R' ayant les significations données ci-dessus.

2. Compositions selon la revendication 1, dans lesquelles X, dans la formule générale (I), est choisi parmi hydrogène, les groupes alcoxy en C₈-C₂₀ et alkyle en C₁-C₄, en particulier, parmi hydrogène, méthyle et alcoxy en C₁₀-C₁₈.

3. Compositions selon l'une quelconque des revendications précédentes, dans lesquelles R, dans la formule générale (I), est choisi parmi hydrogène et alkyle en C₁-C₂₀, en particulier, parmi hydrogène, méthyle et éthyle.

4. Compositions selon l'une quelconque des revendications précédentes, dans lesquelles R' est choisi parmi alkyle en C₁-C₂₀ et les groupes de formule générale (II), dans laquelle R₁ représente alkyle en C₁-C₂₀, en particulier, alkyle en C₉-C₂₀.

5. Compositions selon l'une quelconque des revendications 1 à 4, dans lesquelles les additifs de formule générale (I) ont été synthétisés par réaction de la résorcine, facultativement substituée en position 2, avec un aldéhyde, et estérification ultérieure du produit résultant avec un halogénure de formule R'-Y, où R' a les significations données à la revendication 1 et Y représente halogène, ou avec un anhydride d'acide.

6. Compositions selon l'une quelconque des revendications 1 à 5, dans lesquelles les additifs de formule générale (I) ont été ajoutés aux fractions hydrocarbonées en tant que tels ou dissous dans un solvant.

7. Compositions selon l'une quelconque des revendications précédentes, dans lesquelles les additifs ont été ajoutés aux fractions hydrocarbonées dans des quantités d'au moins 0,001% en poids, de préférence, de 0,001 à 1, et en particulier, de 0,005 à 0,15% en poids.

8. Compositions selon l'une quelconque des revendications précédentes, montrant une diminution du Point de Trouble allant jusqu'à 3°C et une diminution du Point d'Ecoulement d'au moins 10°C, par comparaison avec les compositions correspondantes sans additif.

9. Procédé de fabrication des compositions d'hydrocarbures telles que définies à l'une quelconque des revendications précédentes, comprenant l'addition d'au moins un additif de formule générale (I) à un Distillat Moyen, dans une quantité d'au moins 0,001% en poids.
